# EUROPEAN PATENT SPECIFICATION

(11) **EP 1 427 370 B1**
(45) Date of publication and mention of the grant of the patent: **03.10.2012**
(21) Application number: 01961508.7
(22) Date of filing: 21.08.2001
(51) Int. Cl.: A61F 13/64, A61F 13/15, A61F 13/56, A61F 13/49

(54) **ABSORBENT ARTICLE WITH FOLDED ARCUATE SHAPED BELT AND METHOD FOR ITS MANUFACTURE**
SAUGFÄHIGER ARTIKEL MIT GEFALTETEM BOGENFÖRMIGEM GÜRTEL UND HERSTELLUNGSMETHODE DAFÜR
ARTICLE ABSORBANT A CEINTURE CINTREE REPLIEE, ET SON PROCEDE DE FABRICATION

(30) Priority: 18.09.2000 SE 0003331
(43) Date of publication of application: 16.06.2004
(73) Proprietor: SCA Hygiene Products AB, 405 03 Göteborg (SE)
(72) Inventor: KUSIBOJOSKA, Liljana, 254 39 Helsingborg (SE); HERMANSSON, Kent, 426 54 Vastra Frolunda (SE)
(74) Representative: Egeröd, Lisbeth
(86) International application number: PCT/SE2001/001777
(87) International publication number: WO 2002/022062

(56) References cited:
- WO-A1-91/08725
- WO-A1-95/29657
- JP-A- 9 253 123
- JP-A- 10 314 228
- US-A- 1 614 239
- US-A- 6 051 094
- PATENT ABSTRACTS OF JAPAN & JP 09 253 123 A (UNI CHARM CORP) 30 September 1997
- PATENT ABSTRACTS OF JAPAN & JP 10 314 228 A (TOYO EIZAI KK) 02 December 1998

## Description

### Technical field

The present invention refers to an absorbent article such as a diaper and an incontinence guard comprising a liquid permeable topsheet, a liquid impermeable backsheet and an absorbent body enclosed therebetween, said article having a front portion, a rear portion and a crotch portion therebetween, and further is provided with a pair of belt members attached to the rear portion, alternatively the front portion, of the article and which are intended to be fastened together around the waist of the wearer and where said front portion, alternatively said rear portion, is provided with fastening means intended to be fastened to the belt members, in such a way that the article will assume a pantlike shape, where the belt members form a part of the waist portions of the pant. The invention also refers to a method for manufacturing the article.

### Background of the invention

Diapers and incontinence guards for incontinent adults usually have a garment portion holding an absorbent body in place against the user's body and attachment means which hold the garment portion in place also when the user is moving. A common type of attachment means are adhesive tapes or hook and loop fasteners of the touch-and-close type which directly attach the front and rear portions of the absorbent article to each other. It is further known, through e g EP-A-0 287 388, EP-A-0 409 307, EP-A-0 605 012 and FR-A-2 586 558, to attach the front and rear portions of the article by means of a belt, at which the possibilities to adjust the fit are improved. The belt further provides a simplified change of diaper or incontinence guard, especially when the wearer is standing.

The ends of the belt members can however be difficult to grasp when the article shall be applied to a wearer, especially if the wearer is lying down and the belt ends get caught under the wearer. Besides the long belt ends may cause processability problems during the manufacturing of the article. It is therefore a desire that the belt ends before use are folded and gathered at an easily accessible location on the article.

The belt members are usually straight. It has however proven that a belt having an arcuate shape sometimes can give an improved fit around the waist of the wearer. Such an arcuate belt is known through WO 91/08725. This document does however not give any solution to the problem of prefolding the belt members during manufacture. Through WO 86/04812 it is previously known to fold the attachment straps of a diaper, i e the straps that carry the attachment means that fasten up the front and rear portions of the diaper, in accordion-like fashion before they are applied to the diaper in order to simplify manufacture and packaging of the products. The folded attachment straps are kept together by a weak adhesive applied in narrow zones in order to be easily released and unfolded when the diaper is ready for use. The attachment straps are straight and do not constitute a belt as defined in the present invention.

### Object and most important features of the invention

The object of the present invention is to accomplish a belt-provided absorbent article, such as a diaper or incontinence guard as defined in claim 1.

According to a preferred embodiment the belt members are held in place in their double-folded position against the rear portion of the article by means of an easily breakable seal.

The invention also refers to a method for manufacturing a belt-provided article, at which the belt portions before they are applied to the article are folded double and then cut in an arcuate shape, where the tangent to the arcuate curve formed by the respective longitudinal side edges of the belt members, in the area of the fold make a substantially right angle to the fold, and that the belt members in their double folded position are applied to the rear portion of the article.

### Description of drawings

The invention will in the following be closer described with reference to an embodiment shown in the accompanying drawings.
Fig. 1 shows schematically a perspective view of a diaper or incontinence guard according to the invention having the belt portions folded.
Fig. 2 shows the diaper according to Fig. 1 but having the belt portions extended and unfolded.
Fig. 3 shows schematically the manufacture of the belt members by cutting a double-folded piece of material.

### Description of an embodiment

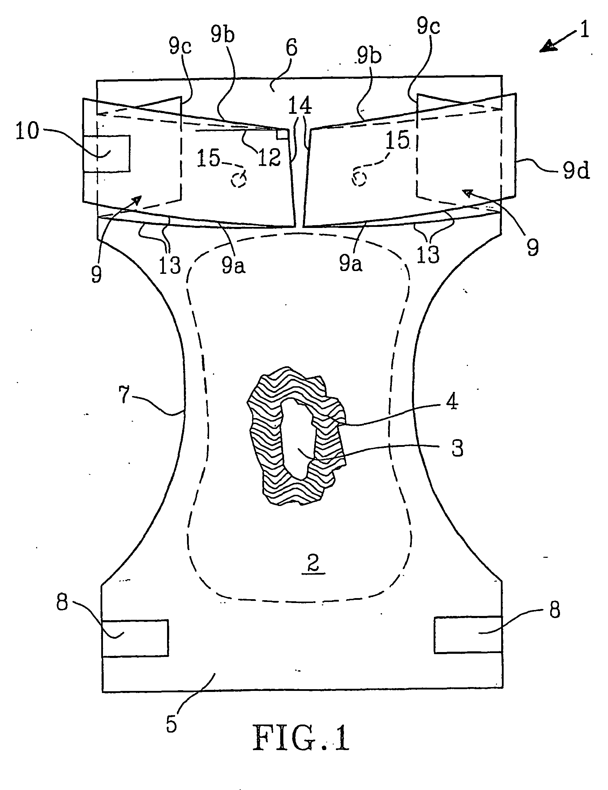

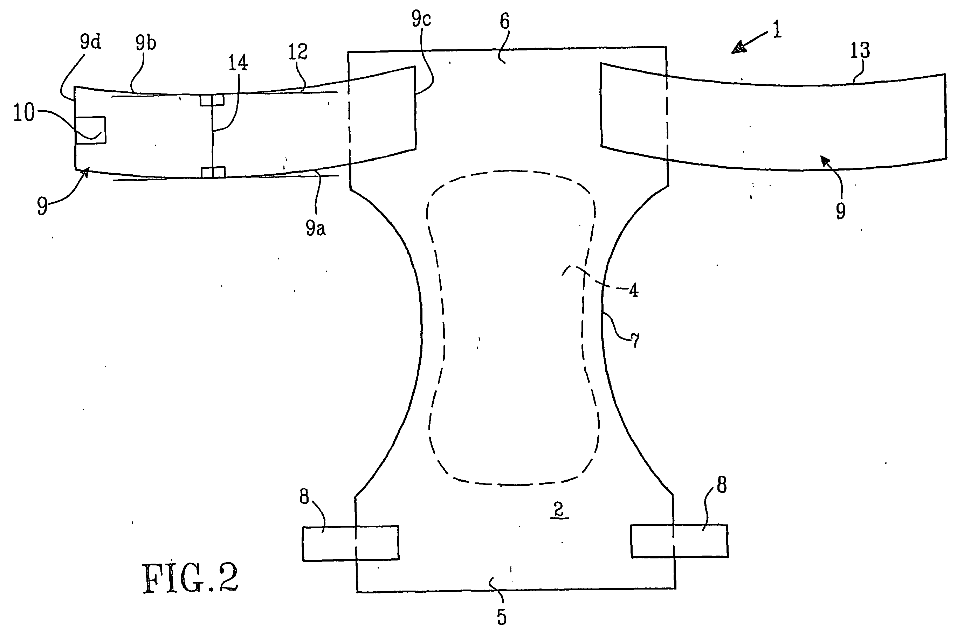

Figs. 1 and 2 shows an embodiment of a diaper or incontinence guard 1 comprising a liquid permeable topsheet 2, a liquid impermeable backsheet 3 and an absorbent body 4 enclosed therebetween. The liquid permeable topsheet 2 can consist of a nonwoven material, e g a spunbond material of continuous filaments, a meltblown material or a bonded carded fibrous web. The liquid impermeable backsheet 3 may consist of a plastic film, a nonwoven material coated with a liquid impervious material or a hydrophobic nonwoven material which resists liquid penetration.

The topsheet 2 and the backsheet material 3 has a somewhat greater extension in the plane than the absorbent body 4 and extends outside the edges thereof. The layers 2 and 3 are connected to each other within the projecting portions thereof, e g by gluing or welding by heat or ultrasonic.

The absorbent body 4 can be of any conventional kind. Examples of commonly occurring absorbent materials are cellulosic fluff pulp, tissue layers, highly absorbent polymers (so called superabsorbents), absorbent foam materials, absorbent nonwovens or the like. It is common to combine cellulosic fluff pulp with superabsorbents in an absorbent body. It is also common to have absorbent bodies comprising layers of different material with different properties with respect to liquid acquisition capacity, liquid distribution capacity and storage capacity. It is well-known to the person skilled in the art and does therefore not have to be described in detail. The thin absorbent bodies which are common in for example baby diapers and incontinence guards often comprise a compressed mixed or layered structure of cellulosic fluff pulp and superabsorbent.

The diaper/incontinence guard is intended to enclose the lower part of the wearer's trunk like a pair of absorbent pants. It comprises a front portion 5 intended during use to be worn on the front part of the user's body, a rear portion 6 intended during use to be worn on the rear part of the user's body, and a more narrow crotch portion 7 located between the front and rear portions and which is intended to be worn in the crotch part of the user between the legs. The front portion 5 is provided with a pair of adhesive tape portions 8 or other type of attachment means such as hooks and loops fasteners of the touch-and-close type.

A pair of belt members 9 are with one end attached, e g glued or ultrasonically welded, to the rear portion 5 of the diaper. The belt members 9 are with their opposite ends intended to be fastened together, e g by means of tape tab 10 which is taped against the outside of the opposite belt member. Instead of a tape tab there may be another optional attachment means, such as hook-and-loop type fasteners. The attachment tapes 8 of the front portion 5 or corresponding attachment means are intended to be attached against the outsides of the belt members 9 in order to fasten together the diaper/incontinence guard to the desired pantlike shape. The belt members have opposed longitudinal side edges 9a,b and end edges 9c,d, at which one end edge 9c is attached to the rear portion of the diaper and the other end edge 9d of one belt portion carries said fastening means 10.

The width of the belt members 9 should be between 5-20 cm, preferably between 7-15 cm.

The belt members 9 are preferably a laminate of a carrier material, which forms the outside of the belt, and a soft nonwoven, which forms the inside of the belt intended to be in direct contact with the skin of the user.

A suitable nonwoven material can be a spunbond material of e g polypropylene- or polyethylene fibres. Conjugate fibres may also be used. Another suitable nonwoven material can be a carded thermobonded material of e g polypropylene- , polyester- or conjugate fibres.

As a carrier material there can be used a plastic film or another appropriate material, e g a nonwoven. The carrier material should be adapted to function as a reception surface for the attachment means 8 and 10, at which in those cases the attachment means are tape tabs, a plastic film is suitable. In case other types of attachment means are used instead of tape tabs, e g hook-and-loop type fasteners, another type of carrier material is suitable which may function as a reception surface for the attachment means in question. Also elastic laminates are suitable to use as material in the belt portions.

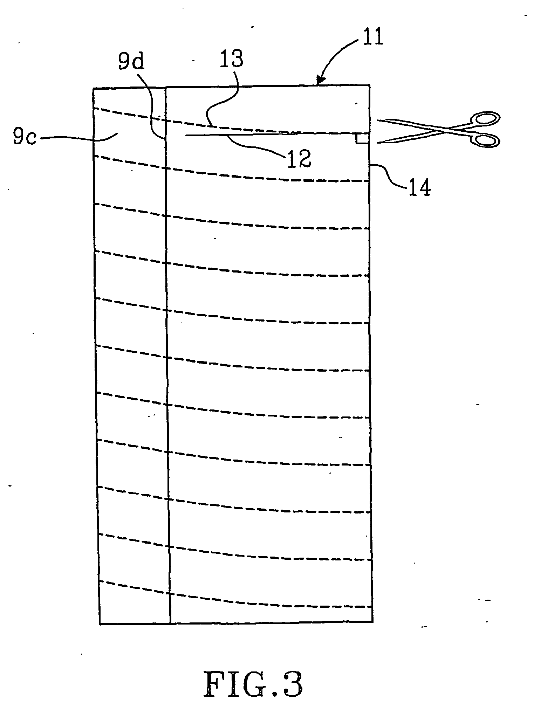

The belt members 9 are before use double folded and cut in the shape of half an arch. This is made, as is shown in Fig. 3, by folding a piece of material, which is intended to form the belt members 9, together and then cutting the double folded material in the shape of half an arch, in such a way that the tangent 12 to the arcuate curve 13 formed by the respective cutting line in the area of the fold 14 make a substantially right angle to the fold. Said cutting lines will form the longitudinal side edges 9a and b of the belt members. By this the belt members will in their unfolded extended position form an arch-shape where the portions on opposite sides of the fold 14 are the mirror image of each other. A plurality of belt members can be cut from the same piece of material 11 without waste of material therebetween. Preferably the fold 14 is placed so that the belt member on one side of the fold 14 will be longer than on the other side of the fold. The longer projecting end can by this make the end 9c which is attached to the rear portion 6 of the diaper. It is also possible, in case a long belt is desired, to place the fold 14 so that the belt members are equally long on both sides of the fold or even so that the longer projecting end makes the end 9d which is to be attached to the opposite belt member. By this said end 9d will project outside the side edges of the rear portion 6 or alternatively be folded back over this.

The belt members 9 are in their folded position with one end edge 9c and the portions adjacent thereto attached to the rear portion 6 of the diaper in a known manner. The belt members 9 are then folded in towards the rear portion 6 and preferably attached in this position by an easily breakable seal 15, such as a glue string/glue dot or welding seam/welding dot provided by ultrasonic or heat.

When the diaper/incontinence guard is to be applied on the wearer the belt members 9 are easily accessible to the nurse and by a light jerk in the belt end 9d the seal 15 is broken and the belt members 9 can be unfolded and fastened together around the waist of the wearer. The belt members 9 have as shown in Fig. 2 an arcuate shape, which gives an improved fit around the waist of the wearer. The fastening means 8 on the front portion 5 can then be attached to the outside of the belt members 9 in order to fasten together the diaper/incontinence guard to the desired pantlike shape.

The invention is of course not limited to the above described embodiment but can be modified within the scope of the claims.

## Claims

1. Absorbent article such as a diaper and an incontinence guard comprising a liquid permeable topsheet (2), a liquid Impermeable backsheet (3) and an absorbent body (4) enclosed therebetween, said article having a front portion (5), a rear portion (6) and a crotch portion (7) therebetween, and further is provided with a pair of belt members (9) attached to the rear portion (6), alternatively to the front portion, of the article and which are intended to be fastened together around the waist of the wearer and where said front portion (5), alternatively said rear portion, is provided with fastening means (8) intended to be fastened to the belt members (9), in such a way that the article will assume a pantlike shape, where the belt members (9) form a part of the waist portions of the pant,
**characterized in**
**that** the belt members (9) before use are in a double-folded condition and have an arcuate shape in which the tangent (12) to the arcuate curve (13) formed by the respective longitudinal edges (9a,b) of the belt members (9) in the area of the fold (14) makes a substantially right angle to the fold, and that they when the article is to be used on a wearer are intended to be unfolded, at which the belt members in their unfolded position form an arcuate shape where the portions on either sides of the fold (14) are the mirror image of each other.

2. Absorbent article as claimed in claim 1,
**characterized in**
**that** the belt members (9) are kept in place in their double folded position against the rear portion (6) of the article by means of an easily breakable seal (15).

3. Method for manufacturing an absorbent article such as a diaper and an incontinence guard comprising a liquid permeable topsheet (2), a liquid impermeable backsheet (3) and an absorbent body (4) enclosed therebetween, said article having a front portion (5), a rear portion (6) and a crotch portion (7) therebetween, and further is provided with a pair of belt members (9) attached to the rear portion (6) of the article and which are intended to be fastened together around the waist of the wearer and where said front portion (5) is provided with fastening means (8) intended to be fastened to the belt members (9), in such a way that the article will assume a pantlike shape, where the belt members (9) form a part of the waist portions of the pant,
**characterized in**
**that** the belt members (9) before being applied to the article are double folded and then cut in an arcuate shape in which the tangent (12) to the arcuate curve (13) formed by the respective longitudinal side edges of the belt portions in the area of the fold (14) makes a substantially right angle to the fold (14), and that the belt members (9) in their double folded position are applied to the rear portion (6) of the article.

4. Method as claimed in claim 3,
**characterized in**
**that** the belt members (9) are attached to the rear portion (6) of the article in their double folded position by means of an easily breakable seal (15).

## Patentansprüche

1. Saugfähiger Artikel, wie zum Beispiel eine Windel und ein Inkontinenzschutz, mit einer flüssigkeitsdurchlessigen oberen Schicht (2), einer flüssigkeitsundurchlässigen hinteren Schicht (3) und einem dazwischen eingeschlossenen Körper (4), wobei der Artikel einen vorderen Abschnitt (5), einen hinteren Abschnitt (6) und dazwischen einen Schrittabschnitt (7) aufweist und ferner mit einem Paar Gürtelelemente (9) bereitgestellt ist, die an dem hinteren Abschnitt (6), alternativ an dem vorderen Abschnitt, des Artikels angebracht sind und die vorgesehen sind, aneinander um die Taille des Trägers befestigt zu werden, und wobei der vordere Abschnitt (5), alternativ der hintere Abschnitt, mit einem Befestigungsmittel (8) bereitgestellt ist, das vorgesehen ist, um an den Gürtelelementen (9) befestigt zu werden, und zwar so, dass der Artikel eine hosenartige Form annehmen wird, bei der die Gürtelelemente (9) einen Teil des Taillenbereichs der Hose ausbilden,
**dadurch gekennzeichnet,**
**dass** die Gürtelelemente (9) vor Verwendung in einem doppelt gefalteten zustand sind und eine bogenförmige Form aufweisen, in der die Tangente (12) zu der bogenförmigen Kurve (13), die durch die jeweiligen Längskanten (9a, b) der Gürtelelemente (9) in dem Bereich der Faltung (14) ausgebildet ist, einen im Wesentlichen rechten Winkel zu der Faltung erzeugt, und dass sie, wenn der Artikel an einem Träger verwendet wird, vorgesehen sind, entfaltet zu werden, bei dem die Gürtelelemente in ihrer entfalteten Position eine bogenförmige Form ausbilden, wobei die Abschnitte an jeder Seite der Faltung (14) zueinander spiegelbildlich sind.

2. Saugfähiger Artikel nach Anspruch 1, **dadurch gekennzeichnet,**
**dass** die Gürtelelemente (9) mittels eines einfach aufzubrechenden Siegels (15) in ihrer doppelt gefalteten Position gegen den hinteren Abschnitt (6) des Artikels gehalten werden.

3. Verfahren zum Herstellen eines saugfähigen Artikels, wie zum Beispiel einer Windel und einem Inkontinenzschutz, mit einer flüssigkeitsdurchlässigen oberen Schicht (2), einer flüssigkeitsundurchlässigen hinteren Schicht (3) und einem dazwischen eingeschlossenen saugfähigen Kern (4), wobei der Artikel einen vorderen Abschnitt (5), einen hinteren Abschnitt (6) und dazwischen einen Schrittabschnitt (7) aufweist und ferner mit einem Paar Gürtelelemente (9) bereitgestellt ist, die an dem hinteren Abschnitt (6) des Artikels angebracht sind und die vorgesehen sind, um aneinander um die Taille des Trägers befestigt zu werden und wobei der vordere Abschnitt (5) mit Befestigungselementen (8) bereitgestellt ist, die vorgesehen sind, um an den Gürtelelementen befestigt zu werden, sodass der Artikel eine hosenartige Form annehmen wird, wobei die Gürtelelemente (9) einen Teil des Taillenabschnitts der Hose ausbilden,
**dadurch gekennzeichnet,**
**dass** die Gürtelelemente (9) vor dem Aufbringen auf den Artikel doppelt gefaltet werden und dann in einer bogenförmigen Form geschnitten werden, in der die Tangente (12) zu der bogenförmigen Kurve (13), die durch die jeweiligen seitlichen Längskanten der Gürtelabschnitte in dem Bereich der Faltung (14) ausgebildet ist, einen im Wesentlichen rechten Winkel zu der Faltung (14) erzeugt, und dass die Gürtelelemente (9) in ihrer doppelt gefalteten Position auf den hinteren Abschnitt (6) des Artikels aufgebracht werden.

4. Verfahren nach Anspruch 3,
**dadurch gekennzeichnet,**
**dass** die Gürtelelemente (9) in ihrer doppelt gefalteten Position mittels eines einfach aufzubrechenden Siegels (15) an dem hinteren Abschnitt (6) des Artikels angebracht werden.

## Revendications

1. Article absorbant tel qu'une couche-culotte et une protection contre l'incontinence comprenant une feuille supérieure (2) perméable aux liquides, une feuille arrière (3) imperméable aux liquides et un corps absorbant (4) enfermé entre celles-ci, ledit article comportant une partie avant (5), une partie arrière (6) et une partie entrejambe (7) située entre celles-ci, et ledit article étant doté en outre d'une paire d'éléments de ceinture (9) qui sont fixés à la partie arrière (6) de l'article, en variante à la partie avant, de l'article et qui sont destinés à être attachés l'un à l'autre autour de la taille de l'utilisateur, et ladite partie avant (5), en variante ladite partie arrière étant munie de moyens d'attache (8) destinés à être attaché aux éléments de ceinture (9) de telle manière que l'article prenne une forme analogue à celle d'une culotte, les éléments de ceinture (9) constituant une portion des parties taille de la culotte,
**caractérisé en ce que**
les éléments de ceinture (9) sont à l'état doublement replié avant l'utilisation et ont une forme courbe dans laquelle la tangente (12) à la courbe arquée (13) formée par les bords longitudinaux respectifs (9a, b) des éléments de ceinture (9) dans la zone du pli (14) forme un angle substantiellement droit par rapport au pli et **en ce qu'**ils sont destinés à être dépliés lorsque l'article doit être utilisé sur un utilisateur, sur lequel les éléments de ceinture, dans leur état déplié, ont une forme courbe dans laquelle les parties situées des deux côtés du pli (14) sont symétriques l'une de l'autre.

2. Article absorbant selon la revendication 1 **caractérisé en ce que**
les éléments de ceinture (9) sont maintenues en place dans leur état doublement replié contre la partie arrière (6) de l'article au moyen d'un sceau (15) qui peut être facilement rompu.

3. Procédé de fabrication d'un article absorbant tel qu'une couche-culotte et une protection contre l'incontinence comprenant une feuille supérieure (2) perméable aux liquides, une feuille arrière (3) imperméable aux liquides et un corps absorbant (4) enfermé entre celles-ci, ledit article comportant une partie avant (5), une partie arrière (6) et une partie entrejambe (7) située entre celles-ci et étant muni en outre d'une paire d'éléments de ceinture (9) qui sont fixés à la partie arrière (6) de l'article et qui sont destinés à être attachés l'un à l'autre autour de la taille de l'utilisateur et ladite partie avant (5) étant munie de moyens d'attache (8) destinés à être fixé aux éléments de ceinture (9) de telle manière que l'article prenne une forme analogue à celle d'une culotte, les éléments de ceinture (9) constituant une portion des parties taille de la culotte,
**caractérisé en ce que**
les éléments de ceinture (9) sont doublement repliés avant d'être appliqués sur l'article, puis coupés selon une forme courbe dans laquelle la tangente (12) à la forme courbe (13) formée par les bords latéraux longitudinaux respectifs des parties ceinture dans la zone du pli (14) forment un angle substantiellement droit par rapport au pli (14) et **en ce que** les éléments de ceinture (9) sont appliqués sur la partie arrière (6) de l'article à l'état doublement replié.

4. Procédé selon la revendication 3
**caractérisé en ce que**
les éléments de ceinture (9) sont attachés à l'état doublement replié à la partie arrière (6) de l'article à l'aide d'un sceau (15) qui peut être facilement rompu.
